# FASCICULE DE BREVET EUROPEEN

(11) **EP 0 462 031 B1**
(45) Date de publication et mention de la délivrance du brevet: **10.08.1994**
(21) Numéro de dépôt: 91420185.0
(22) Date de dépôt: 07.06.1991
(51) Int. Cl.: C07C 41/06, C07C 43/15

(54) **Procédé de préparation de dialcoxybutènes**
Verfahren zur Herstellung von Dialkoxybutenen
Method for the production of dialkoxybutenes

(30) Priorité: 13.06.1990 FR 9007608
(43) Date de publication de la demande: 18.12.1991
(73) Titulaire: RHONE-POULENC CHIMIE, 92408 Courbevoie Cédex (FR)
(72) Inventeur: Laucher, Dominique, F-69007 Lyon (FR); Costantini, Michel, F-69003 Lyon (FR)
(74) Mandataire: Vignally, Noel

(56) Documents cités:
- EP-A- 0 042 097
- DE-A- 2 407 898
- GB-A- 1 138 366
- US-A- 4 533 761
- US-A- 4 843 180

## Description

La présente invention a pour objet un procédé de préparation de dialcoxybutènes. Elle concerne plus particulièrement un procédé de préparation de dialcoxybutènes à partir du butadiène -1,3 et d'un alcool.

Dans GB-A- 1 138 366 il a été proposé de préparer le diméthoxy-1,4 butène-2 par réaction du butadiène-1,3 et du méthanol en présence d'une solution de chlorure palladeux et de chlorure cuivrique dihydraté. Il est également suggéré de conduire ce type de réactions en phase liquide en présence de sels autres que les nitrates et les sulfates ou de composés de coordination du palladium, du platine, du nickel, du fer ou du cobalt et d'un système rédox minéral. Toutefois l'efficacité de ce procédé en phase homogène reste faible ; le rendement en diméthoxybutènes est insuffisant et la présence d'une quantité importante de sous-produits peut-être détectée.

Il a été aussi cité dans EP-A- 42 097 un procédé pour la préparation d'esters de butène diol en présence d'un catalyseur à base de palladium et de tellure sans ou avec cuivre, sur charbon actif.

Il a également été proposé dans US-A-4,533,761 de préparer des dialcoxybutènes par réaction du butadiène-1,3 et d'un alcanol, l'alcanol étant le milieu réactionnel, en présence d'ions iodure et cuivre.

Toutefois, la réaction paraît stoechiométrique par rapport au cuivre et on constate la formation non désirée de divers sous-produits.

Il a maintenant été trouvé un procédé de préparation de dialcoxybutènes, à partir du butadiène-1,3 et d'un alcool, ne présentant pas les inconvénients précités.

La présente invention a donc pour objet un procédé de préparation de dialcoxybutènes par réaction du butadiène-1,3 et d'un alcool en présence d'un catalyseur caractérisé en ce que le catalyseur est un solide comprenant au moins un premier composant choisi parmi les métaux nobles du groupe VIII de la classification périodique des éléments et au moins un second composant choisi parmi le tellure, le sélénium et le soufre, disposés sur un support et en ce que la réaction es conduite en présence d'oxygène.

Les dialcoxybutènes sont des intermédiaires utiles notamment à la préparation de composés du furane. Par dialcoxybutènes on entend dans le cadre du présent procédé le dialcoxy-1,4 butène-2 et le dialcoxy-1,2 butène-3 dont les groupes alcoxy linéaires, branchés ou cycliques comportent de 1 à 12 atomes de carbone et, de préférence, de 1 à 4 atomes de carbone. Ces diéthers peuvent être préparés selon l'invention par réaction du butadiène-1,3 et d'un alcool, ledit alcool pouvant constituer le milieu de réaction. On utilise un alcool linéaire, branché ou cyclique, comportant de 1 à 12 atomes de carbone et, de préférence, de 1 à 4 atomes de carbone. Cet alcool ainsi que le butadiène-1,3 peuvent être de qualité technique.

A titre d'exemples d'alcools susceptibles de convenir à la mise en oeuvre du présent procédé, on peut citer : le méthanol, l'éthanol, l'isopropanol, le tertiobutanol, l'alcool benzylique, le phényl-2 éthanol et le trifluoroéthanol. Conviennent plus particulièrement le méthanol et l'éthanol.

Selon la présente invention, on met en oeuvre un catalyseur solide comprenant au moins un premier composant choisi parmi les métaux nobles du groupe VIII de la classification périodique des éléments et au moins un second composant choisi parmi le tellure, le sélénium et le soufre, disposés sur un support.

Le catalyseur peut contenir comme premier composant plus particulièrement du palladium, du rhodium ou du platine et, on recourt avantageusement à des catalyseurs à base de palladium.

Le support utilisé pour la préparation du catalyseur n'est pas un facteur essentiel. Sont susceptibles de convenir à cet effet, notamment, les charbons actifs, les gels de silice, les mélanges silice-alumine, l'alumine, les argiles, la bauxite, la magnésie et la terre de diatomées.

Pour une bonne mise en oeuvre de la présente invention on recourt aux charbons actifs.

La quantité de composant(s) choisi(s) parmi les métaux nobles du groupe VIII peut varier dans de larges limites. Pour des raisons économiques, la Demanderesse préconise un maximum de concentration d'un tel élément sur le support de l'ordre de 20 % du poids du catalyseur solide, bien que des teneurs plus élevées puissent être utilisées.

Pour obtenir une vitesse de réaction acceptable, il est préférable que cette concentration soit d'au moins 0,1 % en poids.

Pour une bonne mise en oeuvre du procédé selon l'invention le catalyseur solide comprend également au moins un composant additionnel choisi dans le groupe constitué par le bismuth, l'antimoine, le cuivre et l'étain.

Bien que la quantité de tels composants additionnels sur le support comme celle du (ou des) seconds composant(s) puisse varier dans de larges limites elles représentent en général et au total de 0,03 à 30 % en poids du catalyseur.

Parmi les les seconds composants en cause on préfère le tellure. Le tellure peut être présent isolément ou en combinaison avec l'un au moins des éléments additionnels choisi dans le groupe constitué par le cuivre, l'étain et l'antimoine.

Généralement le rapport molaire entre chacun des éléments seconds ou additionnels et le (ou les) métal (métaux) noble(s) du groupe VIII est compris entre 0,01 et 10 et, de préférence entre 0,1 et 5.

De bons résultats peuvent être obtenus avec le couple palladium-tellure, dans les rapports molaires précités, disposé sur du charbon actif et, le cas échéant, associé à du cuivre, de l'étain ou de l'antimoine.

Pour préparer le catalyseur supporté utile à la mise en oeuvre du procédé selon la présente invention, on peut recourir à des techniques classiques, connues en elles-mêmes, de préparation de catalyseurs métalliques supportés.

Le catalyseur peut être préparé, par exemple en introduisant un support dans une solution que l'on prépare en dissolvant au moins un composé approprié du(ou des) élément(s) choisi(s) ; le dépôt du ou des composants actifs sur le support est réalisé en distillant le solvant et la masse de contact ainsi obtenue est soumise à une réduction au moyen d'un courant d'hydrogène ou par un composé réducteur tels l'hydrazine, le méthanol et la formaline.

Selon un autre mode de préparation classique le dépôt du ou des composants actifs sur le support est réalisé en précipitant les composés de manière en soi connue et soumettant la masse de contact ainsi obtenue à une réduction.

D'autres modes de préparation sont également possibles, en particulier l'imprégnation d'un support au moyen d'une solution du(ou des) composés appropriés en présence d'un réducteur organique tel l'hydrazine.

Le dépôt sur le support de plusieurs composants peut bien entendu être réalisé simultanément ou séparément.

La nature du composé d'au moins un métal noble du groupe VIII utilisé pour l'ellaboration du catalyseur dans le cadre de la présente invention n'est pas cruciale. De même, la nature précise du ou des composé(s) des autres éléments n'est pas d'une importance essentielle.

Si on le désire, on peut utiliser les métaux eux-mêmes tels le palladium métallique, le rhodium métallique, le tellure métallique, le bismuth métallique et le cuivre métallique.

A titre d'exemples de composés utilisables dans la préparation des catalyseurs en cause on citera : le chlorure de palladium, le chlorure de platine, l'acétate de palladium, l'acétate de platine, le chlorure mixte de sodium et de palladium, le sulfate mixte de sodium et de palladium, l'acide hexachloroplatinique, le nitrate de rhodium, le chlorure d'antimoine, le chlorure de bismuth, le chlorure de tellure (II) ou (IV), le chlorure de sélénium (II) ou (IV), l'oxyde de tellure (IV) ou (VI), l'oxyde de sélénium, l'acétate de cuivre, l'acétate d'antimoine, le nitrate de bismuth et le chlorure de bismuth.

La dimension de particules de la masse de contact se situe en général entre 0,1 et 10 mm, étant entendu que des dimensions plus grandes ou plus petites peuvent être choisies. La dimension précisément retenue dépend de manière en soi-connue de la conduite opératoire choisie.

Comme indiqué en tête du présent mémoire, les dialcoxybutènes sont obtenus par réaction du butadiène-1,3 et d'un alcool en présence d'un catalyseur solide, défini ci-avant, et d'oxygène.

La réaction est avantageusement conduite en phase liquide, en mettant en suspension le catalyseur solide dans le milieu réactionnel comprenant un alcool et en y insufflant du butadiène-1,3 et un gaz renfermant de l'oxygène moléculaire. Ce gaz peut être de l'oxygène pur ou de l'oxygène dilué avec un gaz inerte, par exemple de l'air. La quantité d'oxygène ne présente de caractère critique que dans la mesure où elle devra être telle que ni les gaz d'alimentation, ni une éventuelle phase gazeuse susceptible d'apparaître dans la zone réactionnelle se trouve dans la plage des compositions explosives, compte-tenu des autres paramètres ou conditions réactionnelles choisis. La quantité d'oxygène peut être en excès ou en défaut par rapport à la stoéchiométrie de réaction, vis-à-vis du butadiène.

La quantité de métal noble du groupe VIII peut varier dans de large limites par rapport au butadiène. Cette quantité est en général comprise (sur une base molaire) entre 10⁻⁵ et 10⁻² et, de préférence entre 10⁻⁴ et 10⁻³.

La pression partielle d'oxygène mesurée à 25°C est de préférnce comprise entre 0,1 et 20 bar.

La température de réaction est généralement comprise entre 20 et 160°C et, de préférence entre 70 et 120°C. En dessous de 70°C la vitesse de réaction est relativement lente et au-delà de 120°C on peut observer une décomposition des réactifs et des produits.

Selon le mode opératoire, la pression de réaction varie entre la pression atmosphérique et environ 300 bar. On préfère une pression comprise entre 1 et 100 bar.

En fin de réaction ou du temps imparti à celle-ci, on récupère les dialcoxybutènes recherchés par tout moyen approprié, par exemple, par distillation et/ou extraction, le cas échéant, après filtration ou décantation du catalyseur solide.

### EXEMPLE 1 :

a) Traitement du carbone servant de support
   Du carbone activé fourni par la société CECA S.A., de type 3S et de surface BET de 1150 m²/g (100 g) est mis en suspension dans une solution aqueuse d'acide nitrique à 30 % (600 ml). Le mélange est porté à reflux pendant 2 heures. La solution aqueuse est éliminée par filtration et le carbone est lavé à l'eau déminéralisée jusqu'à obtention d'un pH des eaux de lavage égal à 4. Le carbon est séché à 90°C sous pression réduite de 20 mmHg.
b) Préparation du catalyseur renfermant du Palladium et du Tellure
   Du carbone (10 g) ainsi traité est mis en suspension dans une solution aqueuse d'acide nitrique à 30 % (40 ml) dans laquelle de l'acétate de palladium (2 mmoles, 448 mg) et du dioxyde de tellure (0,6 mmole ; 160 mg) ont été dissouts. Le solvant est éliminé par distillation dans un évaporateur rotatif sous pression réduite. Le solide ainsi obtenu est séché à 80°C sous pression réduite (20 mmHg).
   Le catalyseur est réduit pendant 2 heures à 200°C et 2 heures à 400°C par un courant d'azote (60 l/h) saturé en méthanol. Puis il est traité pendant 16 heures à 300°C avec de l'azote (60 l/h) contenant 2 % d'oxygène et à nouveau réduit pendant 2 heures à 200°C et 2 heures à 400°C avec un courant d'azote saturé en méthanol.
c) Méthoxylation
   Du catalyseur (1 g) ainsi préparé est chargé avec du méthanol (30 ml) et du butadiène (80 mmoles ; 4,3 g) dans un autoclave en tantale, d'une capacité interne de 120 ml, agité par secousses. L'autoclave est pressurisé jusqu'à 100 bar avec de l'air à 21 % d'O₂ puis chauffé pendant 12 heures à 90°C. L'autoclave est refroidi puis dégazé et le contenu est dosé par chromatographie en phase gazeuse. On a obtenu 7,7 mmoles de diméthoxy-1,2-butène-3 (DMBE-1,2) et 23 mmoles de cis+ trans diméthoxy-1,4-butène-2 (DMBE-1,4).

### EXEMPLE 2

Un essai a été effectué suivant le mode opératoire de l'exemple n° 1 mais la réaction a été menée pendant 23 heures. On a obtenu 10,2 mmoles de DMBE-1,2 et 30,2 mmoles de cis+trans DMBE-1,4.

### EXEMPLE 3

Le catalyseur a été préparé selon le mode opératoire de l'exemple n° 1 mais en utilisant du carbone 50S (fourni par la société CECA S.A., de surface BET de 1500 m²/g). Ce catalyseur a été mis en réaction suivant le même mode opératoire que dans l'exemple n° 1. On a obtenu 4 mmoles de DMBE-1,2 et 8,5 mmoles de cis+trans DMBE-1,4.

### EXEMPLE 4

### Catalyseur renfermant Pd-Te-Cu

La préparation du catalyseur a été conduite de la même manière que dans l'exemple n° 1 sauf que 4,8 mmoles d'acétate de palladium, 0,4 mmole de bioxyde de tellure et 13,4 mmoles d'acétate de cuivre ont été dissoutes dans la solution aqueuse d'acide nitrique à 30 %. La méthoxylation a été menée de la même manière que dans l'exemple n° 1. On a obtenu 2 mmoles de DMBE-1,2 et 0,2 mmoles de DMBE 1,4.

### EXEMPLE 5

### Catalyseur renfermant Pd-Te-Sn

La préparation du catalyseur a été conduite de la même manière que dans l'exemple 1 sauf que 0,7 mmole d'acétate de palladium, 0,25 mmole de bioxyde de tellure et 0,62 mmole de chlorure stanneux ont été dissoutes dans la solution aqueuse d'acide nitrique à 30 %. La méthoxylation a été menée de la même manière que dans l'exemple n° 2. On a obtenu 1,1 mmole de DMBE-1,2 et 2,4 mmoles de DMBE-1,4.

### EXEMPLE 6

### Catalyseur renfermant Pd-Te-Sb

La préparation du catalyseur a été conduite de la même manière que dans l'exemple 1 sauf que 2 mmoles d'acétate de palladium, 0,7 mmole de bioxyde de tellure et 1,94 mmole d'acétate d'antimoine ont été dissoutes dans la solution aqueuse d'acide nitrique à 30 %. La méthoxylation a été menée de la même manière que dans l'exemple n° 2. On a obtenu 13,2 mmoles de DMBE-1,2 et 30,3 mmoles de DMBE-1,4.

### EXEMPLE 7

Du catalyseur (1 g) préparé selon le mode opératoire décrit dans l'exemple n° 6, du méthanol (30mml) et du butadiène (35 mmoles ; 1,89 g) ont été chargés dans un autoclave de 120 ml agité par secousses. L'autoclave a été pressurisé à 80 kg/cm2 avec de l'air à 21 % d'oxygène et chauffé pendant douze heures à 90°C. L'autoclave est refroidi puis dégazé et le contenu est dosé par chromatographie en phase gazeuse. On a obtenu 9,3 mmoles de DMBE-1,2 et 20 mmoles de cis+trans DMBE-1,4.

### EXEMPLE 8

Un essai a été réalisé avec le catalyseur et selon le mode opératoire décrits dans l'exemple n° 6 à ceci près que le méthanol a été remplacé par une quantité équivalente de propanol-2 (30 ml). On a obtenu 1,8 mmoles de diisopropyloxy-1,2-butène-3 et 9,7 mmoles de cis+trans diisopropyloxy-1,4-butène-2.

### EXEMPLE 9

Un essai a été réalisé avec le catalyseur et selon le mode opératoire décrits dans l'exemple 6, à ceci près que le méthanol a été remplacé par une quantité équivalente de méthyl-2-propanol. On a obtenu 0,4 mmoles de trans di(méthyl-2-propyloxy-2)-1,4-butène-2.

## Revendications

1. Procédé de préparation de dialcoxybutènes par réaction du butadiène-1,3 et d'un alcool en présence d'un catalyseur caractérisé en ce que le catalyseur est un solide comprenant au moins un premier composant choisi parmi les métaux nobles du groupe VIII de la classification périodique des éléments et au moins un second composant choisi parmi le tellure, le sélénium et le soufre, disposés sur un support et en ce que la réaction est conduite en présence d'oxygène.

2. Procédé selon la revendication 1 caractérisé en ce que le catalyseur comprend du palladium, du rhodium ou du platine.

3. Procédé selon la revendication 1 caractérisé en ce que le catalyseur comprend du palladium.

4. Procédé selon l'une quelconque des revendications précédentes caractérisé en ce que le catalyseur comprend en outre au moins un composant additionnel choisi parmi le bismuth, l'antimoine, le cuivre et l'étain.

5. Procédé selon l'une quelconque des revendications précédentes, caractérisé en ce que le catalyseur comprend comme second composant du tellure.

6. Procédé selon la revendication 5, caractérisé en ce que le catalyseur comprend également du cuivre, de l'antimoine ou de l'étain

7. Procédé selon l'une quelconque des revendications précédentes, caractérisé en ce que le support est du charbon actif.

8. Procédé selon l'une quelconque des revendications précédentes, caractérisé en ce que le catalyseur renferme de 0,01 à 20 % en poids de métal noble du groupe VIII.

9. Procédé selon l'une quelconque des revendications précédentes, caractérisé en ce que la teneur totale en second(s) composant(s) et en composant(s) additionnel(s) représente de 0,01 à 30 % du poids du catalyseur solide.

10. Procédé selon l'une quelconque des revendications précédents, caractérisé en ce que le rapport molaire du (ou des) second(s) composant(s) présent(s) dans le catalyseur solide, au métal noble du groupe VIII est compris entre 0,01 et 10 et, de préférence entre 0,1 et 5.

11. Procédé selon l'une quelconque des revendications précédentes, caractérisé en ce que le rapport molaire du(ou des) composant(s) additionnel(s), au métal noble du groupe VIII est compris entre 0,01 et 10, de préférence entre 0,1 et 5.

12. Procédé selon l'une quelconque des revendications précédentes, caractérisé en ce que la temptérature de réaction est comprise entre 20 et 160°C et, de préférence entre 70 et 120°C.

13. Procédé selon l'une quelconque des revendications précédentes, caractérisé en ce que la pression partielle d'oxygène mesurée à 25°C est comprise entre 0,1 et 20 bar.

14. Procédé selon l'une quelconque des revendications précédentes, caractérisé en ce que la réaction est conduite en phase liquide.

## Patentansprüche

1. Verfahren zur Herstellung von Dialkoxybutenen durch Reaktion von 1.3-Butadien und eines Alkohols in Gegenwart eines Katalysators, dadurch gekennzeichnet, daß der Katalysator ein Feststoff ist, der zumindest eine erste Verbindung enthält, ausgewählt aus den Edelmetallen der Grupe VIII des Periodensystems der Elemente, und wenigstens eine zweite Verbindung, ausgewählt aus dem Tellur, dem Selen und dem Schwefel, auf einem Träger angeordnet, und dadurch, daß die Reaktion in Gegenwart von Sauerstoff durchgeführt wird.

2. Verfahren nach Anspruch 1, dadurch gekennzeichnet, daß der Katalysator Palladium, Rhodium oder Platin enthält.

3. Verfahren nach Anspruch 1, dadurch gekennzeichnet, daß der Katalysator Palladium enthält.

4. Verfahren nach einem beliebigen der vorhergehenden Ansprüche, dadurch gekennzeichnet, daß der Katalysator außerdem wenigstens eine zusätzliche Verbindung enthält, ausgewählt aus dem Wismut, dem Antimon, dem Kupfer und dem Zinn.

5. Verfahren nach einem beliebigen der vorgehenden Ansprüche, dadurch gekennzeichnet, daß der Katalysator als zweite Verbindung Tellur enthält.

6. Verfahren nach Anspruch 5, dadurch gekennzeichnet, daß der Katalysator auch Kupfer, Antimon oder Zinn enthält.

7. Verfahren nach einem beliebigen der vorhergebenden Ansprüche, dadurch gekennzeichnet, daß der Träger Aktivkohle ist.

8. Verfahren nach einem beliebigen der vorhergehenden Ansprüche, dadurch gekennzeichnet, daß der Katalysator 0,01 bis 20 Gewichtsprozent an Edelmetall der Gruppe VIII enthält.

9. Verfahren nach einem beliebigen der vorhergehenden Ansprüche, dadurch gekennzeichnet, daß der Gesamtgehalt an zweiter (oder zweiten) Verbindung(en) und an zusätzlicher (oder zusätzlichen) Verbindung(en) 0.01 bis 30 Gewichtsprozent des festen Katalysators ausmacht.

10. Verfahren nach einem beliebigen der vorhergehenden Ansprüche, dadurch gekennzeichnet, daß das molare Verhältnis der zweiten Verbindung(en) in dem festen Katalysator zu dem Edelmetall der Gruppe VIII zwischen 0,01 und 10 liegt, und vorzugsweise zwischen 0,1 und 5.

11. Verfahren nach einem beliebigen der vorhergehenden Ansprüche, dadurch gekennzeichnet, daß das molare Verhältnis der zusätzlichen Verbindung(en) zu dem Edelmetall der Gruppe VIII zwischen 0,01 und 10 liegt, vorzugsweise zwischen 0.1 und 5.

12. Verfahren nach einem beliebigen der vorhergehenden Ansprüche, dadurch gekennzeichent, daß die Reaktionstemperatur zwischen 20 und 160°C liegt, vorzugsweise zwischen 70 und 120°C.

13. Verfahren nach einem beliebigen der vorhergehenden Ansprüche, dadurch gekennzeichnet, daß der Sauerstoffpartialdruck, bei 25°C gemessen, zwischen 0,1 und 20 bar liegt.

14. Verfahren nach einem beliebigen der vorhergehenden Ansprüche, dadurch gekennzeichnet, daß die Reaktion in flüssiger Phase durchgeführt wird.

## Claims

1. Process for the preparation of dialkoxybutenes by reaction of 1,3-butadiene and an alcohol in the presence of a catalyst, characterized in that the catalyst is a solid comprising at least one first component chosen from noble metals of group VIII of the Periodic Table of the Elements and at least one second component chosen from tellurium, selenium and sulphur, which components are arranged on a support, and in that the reaction is carried out in the presence of oxygen.

2. Process according to claim 1, characterized in that the catalyst contains palladium, rhodium or platinum.

3. Process according to claim 1, characterized in that the catalyst contains palladium.

4. Process according to any one of the preceding claims, characterized in that the catalyst in addition contains at least one additional component chosen from bismuth, antimony, copper and tin.

5. Process according to any one of the preceding claims, characterized in that the catalyst contains tellurium as second component.

6. Process according to claim 5, characterized in that the catalyst also contains copper, antimony or tin.

7. Process according to any one of the preceding claims, characterized in that the support is activated charcoal.

8. Process according to any one of the preceding claims, characterized in that the catalyst contains from 0.01 to 20 % by weight of noble metal of group VIII.

9. Process according to any one of the preceding claims, characterized in that the total content of second component(s) and of additional component(s) represents from 0.01 to 30 % of the weight of the solid catalyst.

10. Process according to any one of the preceding claims, characterized in that the molar ratio of the second component(s) present in the solid catalyst to the noble metal of group VIII is between 0.01 and 10, and preferably between 0.1 and 5.

11. Process according to any one of the preceding claims, characterized in that the molar ratio of the additional component(s) to the noble metal of group VIII is between 0.01 and 10, preferably between 0.1 and 5.

12. Process according to any one of the preceding claims, characterized in that the reaction temperature is between 20 and 160°C, and preferably between 70 and 120°C.

13. Process according to any one of the preceding claims, characterized in that the partial pressure of oxygen measured at 25°C is between 0.1 and 20 bars.

14. Process according to any one of the preceding claims, characterized in that the reaction is carried out in the liquid phase.
